# DEMANDE DE BREVET EUROPEEN

(11) **EP 4 844 779 A2**
(43) Date de publication de la demande: **25.11.2026**
(21) Numéro de dépôt: 26174179.7
(22) Date de dépôt: 23.04.2026
(51) Int. Cl.: G16H 50/30, G16H 40/63, G16H 40/67

(54) **MÉTHODE DE GÉNÉRATION D'ALERTE ET DISPOSITIF ASSOCIÉ**

(30) Priorité: 30.04.2025 FR 2504636
(71) Demandeur: Withings, 92130 Issy-les-Moulineaux (FR)
(72) Inventeur: Plaissy, Anaïs, Issy-les-Moulineaux (FR); Riolacci, Paul-Matthieu, Issy-les-Moulineaux (FR)
(74) Mandataire: Withings IP

(57) **Abrégé**

La présente invention concerne un dispositif de génération d'alerte comprenant une circuiterie de contrôle configuré pour mettre en œuvre une méthode de génération d'alerte, la méthode de génération d'alerte comprenant :
- recevoir des données physiologiques,
- générer une pluralité de partitions en deux sous-intervalles (P1i, P2i) continus,
- pour chaque partition générée, calculer un écart entre une première agrégation (M1i) et une deuxième agrégation (M2i),
- identifier (340) un écart maximum (Δmax) parmi les écarts calculés,
- en réponse à la détermination que la deuxième agrégation (M2) associée à l'écart maximum (Δmax) n'appartient pas à une plage de sensibilité (S), générer un signal d'alerte.

## Description

### Domaine technique

La présente invention concerne une méthode de génération d'alerte, notamment d'une notification envoyée à un utilisateur, sur la base de l'analyse de données d'un paramètre physiologique. La présente invention concerne également un dispositif électronique configuré pour mettre en œuvre une telle méthode.

### Technique antérieure

Il existe divers appareils de mesure connectés, tels que des thermomètres, montres, pèse-personnes et tensiomètres connectés, conçus pour collecter des données relatives à un ou plusieurs paramètres physiologiques d'un utilisateur. Ces dispositifs sont équipés de capteurs et de technologies de communication sans fil qui permettent de transmettre les données recueillies à un serveur, par exemple pour un traitement ultérieur.

L'historique des données collectées par ces appareils est présenté usuellement à l'utilisateur via une application mobile ou un site internet. Cependant, l'interprétation de ces données brutes peut s'avérer complexe pour un utilisateur. En particulier, il est souvent difficile d'identifier des tendances à plus ou moins long terme, de discerner des corrélations entre différents paramètres physiologiques, et de relier ces informations à des aspects concrets de la vie quotidienne de l'utilisateur, tels que ses habitudes de santé, son mode de vie ou un évènement particulier.

Les documents US20210401378A1, WO2017083233A1 et US11089968B2 illustrent des approches visant à analyser les données d'un utilisateur pour identifier des tendances, notamment dans le but de détecter des changements potentiels dans l'état de santé de l'utilisateur et de le notifier en conséquence. Ces documents décrivent des méthodes qui tentent d'extraire des informations significatives à partir des données collectées, afin de fournir des alertes ou des recommandations personnalisées qui pourraient améliorer la gestion de la santé de l'utilisateur.

Toutefois, ces méthodes peuvent être encore améliorées, notamment dans la détection de changement de tendances pertinentes pour l'utilisateur.

### Exposé de l'invention

La présente invention propose une approche qui permet de détecter efficacement un changement de tendance.

A cet effet, la présente description concerne un dispositif de génération d'alerte comprenant une circuiterie de contrôle configurée pour mettre en œuvre une méthode de génération d'alerte, la méthode de génération d'alerte comprenant les étapes suivantes :
- recevoir une série temporelle de données d'un paramètre physiologique d'un utilisateur, dites données physiologiques, les données physiologiques étant associées à un intervalle de temps de détection, chaque donnée physiologique de la série temporelle étant associée à un horodatage inclus dans l'intervalle de temps de détection,
- générer une pluralité de partitions de l'intervalle de temps de détection en deux sous-intervalles continus, avec un premier sous-intervalle suivi d'un deuxième sous-intervalle,
- pour chaque partition générée, calculer un écart entre un résultat d'une fonction d'agrégation appliquée sur les données physiologiques dont l'horodatage est inclus dans le premier sous-intervalle, appelée première agrégation, et un résultat de la fonction d'agrégation appliquée sur les données physiologiques dont l'horodatage est inclus dans le deuxième sous-intervalle, appelée deuxième agrégation,
- identifier un écart maximum parmi les écarts calculés,
- en réponse à la détermination que la deuxième agrégation associée à l'écart maximum n'appartient pas à une plage de sensibilité, générer un signal d'alerte.

La méthode selon la présente description permet de générer un signal d'alerte lorsque la deuxième agrégation sort de la plage de sensibilité. Cette plage de sensibilité est établie de sorte que la deuxième agrégation présente un changement de tendance jugé significatif et que la plage de sensibilité conduise à une génération d'un signal d'alerte pertinent. La plage de sensibilité est notamment déterminée en prenant en compte des facteurs tels que la durée de l'intervalle de temps de détection analysé et/ou le niveau de sensibilité choisi pour l'évaluation. La partition sépare l'intervalle de temps de détection en deux sous-intervalles au niveau d'un marqueur temporel associé notamment à un évènement lié à la santé ou au mode de vie de l'utilisateur.

La méthode inclut l'évaluation de plusieurs partitions qui divise la série temporelle de données en sous-intervalles et la sélection de la partition qui maximise la différence de valeurs d'agrégation entre ces sous-intervalles. Cette approche permet d'identifier la partition la plus pertinente pour détecter des changements de tendance, notamment en maximisant l'écart de valeurs d'agrégation entre les sous-intervalles de la partition et en déterminant un marqueur temporel diviseur entre les deux sous-intervalles qui permet d'identifier évènement dans la vie de l'utilisateur à l'origine de ce changement de tendance. Ainsi, la méthode est capable de mettre en évidence des tendances qui ne seraient pas immédiatement apparentes à l'utilisateur sans une analyse approfondie des données.

Notamment la méthode n'implique pas de traitement lourd des données, comme une entrée massive de données dans un modèle d'intelligence artificielle gourmand en ressources.

Dans un mode de réalisation, la fonction d'agrégation est choisie parmi : une moyenne, une médiane, un écart-type, un maximum, un minimum**.**

Dans un mode de réalisation, le dispositif de génération d'alerte comprend un serveur, un terminal mobile (type téléphone mobile performant) ou un dispositif de mesure (pèse-personne, montre connectée, thermomètre, etc.).

Dans un mode de réalisation, le paramètre physiologique est choisi parmi : une température du poignet, une température corporelle, une fréquence cardiaque nocturne, une fréquence cardiaque au repos, une saturation en oxygène du sang (SpO2), un indice d'apnée-hypopnée, un poids ou une masse, une masse graisseuse (en poids ou en pourcentage), un masse musculaire (en poids ou en pourcentage), une vitesse de l'onde de pouls (VOP, ou PWV), une variabilité de la fréquence cardiaque (VFC, ou HRV en anglais, pour « *heart rate variability* »), un nombre de pas quotidien, une activité sportive (durée, intensité, etc.), une durée du sommeil, un score de qualité de sommeil, une heure du coucher, une heure du lever, la fréquence respiratoire, la pression ou tension artérielle, un niveau d'un composé présent dans l'urine (niveau hormonal, niveau de métabolite, etc.).

Dans un mode de réalisation, l'intervalle de temps de détection présente une durée prédéfinie associée au paramètre physiologique.

Dans un mode de réalisation, chaque intervalle de temps est une fenêtre glissante.

Dans un mode de réalisation, chaque intervalle chaque intervalle de temps a une durée prédéfinie comprise entre 30 jours et 3 ans.

Dans un mode de réalisation, le dispositif de génération d'alerte est configuré pour mettre en œuvre la méthode de génération d'alerte de manière répétée.

Dans un mode de réalisation, le dispositif de génération d'alerte est configuré pour œuvre la méthode de génération d'alerte à chaque réception de donnée, l'intervalle de temps de détection défini comprenant un horodatage associé à la dernière donnée reçue.

Dans un mode de réalisation, l'unité de temps est par exemple une heure, une demi-journée, une journée, une pluralité de jours, une semaine, un mois, etc.

Dans un mode de réalisation, le dispositif est configuré pour effectuer un pré-traitement des données sur chaque unité de temps avant le calcul de l'écart (par exemple pour obtenir HR moyen sur la nuit, un HR par heure, une température moyenne par jour).

Dans un mode de réalisation, chaque sous-intervalle présente une durée minimale.

Dans un mode de réalisation, la durée minimale de chaque sous-intervalle est fonction de la durée de l'intervalle de temps de détection.

Dans un mode de réalisation, la durée minimale de chaque sous-intervalle est fonction du paramètre physiologique.

Dans un mode de réalisation, le dispositif de génération d'alerte est configuré pour définir toutes les partitions possibles de l'intervalle de temps de détection en deux sous-intervalles continus, notamment selon l'unité de temps, et présentant éventuellement une durée minimale de sous-intervalle.

Dans un mode de réalisation, la plage de sensibilité est fonction du paramètre physiologique.

Dans un mode de réalisation, le dispositif de génération d'alerte est en outre configuré pour recevoir une série temporelle de données physiologiques sur un intervalle de temps de sensibilité, chaque donnée physiologique de la série temporelle étant associée à un horodatage inclus dans l'intervalle de temps de sensibilité, la plage de sensibilité étant fonction de l'historique des données du paramètre physiologique sur l'intervalle de temps de sensibilité.

Dans un mode de réalisation, l'intervalle de temps de sensibilité et le deuxième sous-intervalle sont disjoints.

Dans un mode de réalisation, l'intervalle de temps de sensibilité comprend le premier sous-intervalle ou est l'intervalle de temps de détection.

Dans un mode de réalisation, l'intervalle de temps de sensibilité présente une durée plus longue que l'intervalle de temps de détection.

Dans un mode de réalisation, l'intervalle de temps de sensibilité présente une durée prédéfinie associée au paramètre physiologique.

Dans un mode de réalisation, la plage de sensibilité est centrée sur une référence, la référence étant le résultat de ladite fonction d'agrégation appliquée sur des données associées à l'intervalle de temps de sensibilité.

Dans un mode de réalisation, le dispositif de génération d'alerte est configuré pour générer le signal d'alerte si en outre le premier sous-intervalle de la partition est plus grand que le deuxième sous-intervalle.

Dans un mode de réalisation, le dispositif de génération d'alerte est configuré pour générer un message d'alerte à partir du signal d'alerte.

Dans un mode de réalisation, le dispositif de génération d'alerte est configuré pour mettre en œuvre un grand modèle linguistique avec au moins la partition associée à l'écart maximum et les moyennes associées en entrées suite à la génération d'un signal d'alerte pour générer un message d'alerte.

Dans un mode de réalisation, le message d'alerte est destiné à l'utilisateur et/ou un professionnel de santé.

Dans un mode de réalisation, le dispositif de génération d'alerte est configuré pour générer une représentation graphique représentative des deux sous-périodes de la partition associée à l'écart maximum suite à la génération d'un signal d'alerte.

Dans un mode de réalisation, la méthode de génération d'alerte comprend en outre : extraire de la partition associée à l'écart maximum un marqueur temporel diviseur qui partitionne l'intervalle de temps en les deux sous-intervalles continus ; et générer une notification d'évènement temporel, l'évènement temporel étant associé au marqueur temporel diviseur extrait.

Dans un mode de réalisation, le dispositif de génération d'alerte est configuré pour mettre en œuvre un modèle d'apprentissage artificiel avec au moins la partition et les moyennes associées en entrées pour détecter un événement pendant l'intervalle de temps.

Dans un mode de réalisation, l'évènement est une maladie, un événement sportif, une grossesse, un événement festif, des vacances, changement d'habitude de vie, etc.

Dans un mode de réalisation, le dispositif de génération d'alerte est configuré pour générer une nouvelle alerte en réponse à la détermination que, en outre, la durée entre la génération de la précédente alerte et un horodatage associé à la dernière donnée reçu est supérieure à une durée minimale prédéfinie.

Dans un mode de réalisation, le dispositif de génération d'alerte est configuré pour mettre en œuvre la méthode de génération d'alerte pour une pluralité de paramètres physiologiques, notamment sur le même intervalle de temps.

Dans un mode de réalisation, le dispositif de génération d'alerte définit le même intervalle de temps pour chaque paramètre physiologique.

Dans un mode de réalisation, sur la base de la même partition, le dispositif de génération d'alerte est configuré pour calculer l'écart entre une première partition des données d'un paramètre physiologique additionnel associées au premier sous-intervalle avec une deuxième partition des données du paramètre physiologique additionnel associées au deuxième sous-intervalle, identifier un écart maximum parmi les écarts calculés ; identifier un écart maximum ; en réponse à la détermination que la deuxième agrégation associée à l'écart maximum n'appartient pas à une plage de sensibilité, générer un signal d'alerte.

Dans un mode de réalisation, le dispositif de génération d'alerte est configuré pour mettre en œuvre le modèle d'apprentissage artificiel avec au moins la partition et les moyennes associées aux différents paramètres physiologiques en entrées pour détecter un événement pendant l'intervalle de temps.

La présente invention concerne aussi une méthode de génération d'alerte comprenant :
- recevoir une série temporelle de données d'un paramètre physiologique d'un utilisateur, dites données physiologiques, la série temporelle étant associée à un intervalle de temps de détection, chaque donnée physiologique de la série temporelle étant associée à un horodatage inclus de l'intervalle de temps de détection,
- générer une pluralité de partitions de l'intervalle de temps de détection en deux sous-intervalles continus,
- pour chaque partition générée, calculer un écart entre un résultat d'une fonction d'agrégation appliquée sur des données associées au premier sous-intervalle, appelée première agrégation, et un résultat de la fonction d'agrégation appliquée sur des données associées au deuxième sous-intervalle, appelée deuxième agrégation,
- identifier un écart maximum parmi les écarts calculés,
- en réponse à la détermination que la deuxième agrégation associée à l'écart maximum n'appartient pas à une plage de sensibilité, générer un signal d'alerte.

La présente invention concerne un produit programme d'ordinateur comprenant des instructions qui, lorsque le programme est exécuté par une circuiterie de contrôle (notamment le processeur), conduisent celui-ci à mettre en œuvre la méthode de génération d'alerte tel que définie ci-dessus.

### Brève description des dessins

D'autres caractéristiques, détails et avantages apparaîtront à la lecture de la description détaillée ci-après, et à l'analyse des dessins annexés, sur lesquels :
- [Fig. 1] La figure 1 présente un système de suivi selon un mode de réalisation,
- [Fig. 2] La figure 2 présente une vue schématique des composants du système de suivi de la figure 1,
- [Fig. 3] La figure 3 présente un organigramme d'une méthode de génération d'alerte selon un mode de réalisation,
- [Fig. 4] La figure 4 présente quatre exemples (a), (b), (c), (d) de partitions d'un intervalle de temps de détection associé à des données d'un paramètre physiologique,
- [Fig. 5] La figure 5 présente une vue détaillée de l'exemple (b) de la figure 4,
- [Fig. 6] La figure 6 présente un autre exemple de partition d'un intervalle de temps de détection associé à des données d'un autre paramètre physiologique,
- [Fig. 7] La figure 7 présente une vue de l'interface d'utilisateur d'un terminal mobile affichant un message d'alerte,
- [Fig. 8] La figure 8 présente une vue de l'interface d'utilisateur d'un dispositif de mesure affichant un message d'alerte,
- [Fig. 9] La figure 9 présente graphiquement trois mises en œuvre successives d'une méthode de génération d'alerte sur des données de température au poignet d'un utilisateur, et
- [Fig. 10] La figure 10 présente graphiquement la mise en œuvre d'une méthode de génération d'alerte sur des données de poids (a), avec le poids en ordonnée, et sur des données de nombre de pas parcourus par jour (b) d'un utilisateur, avec le nombre en ordonnée.

### Description détaillée des modes de réalisation

### Système de suivi

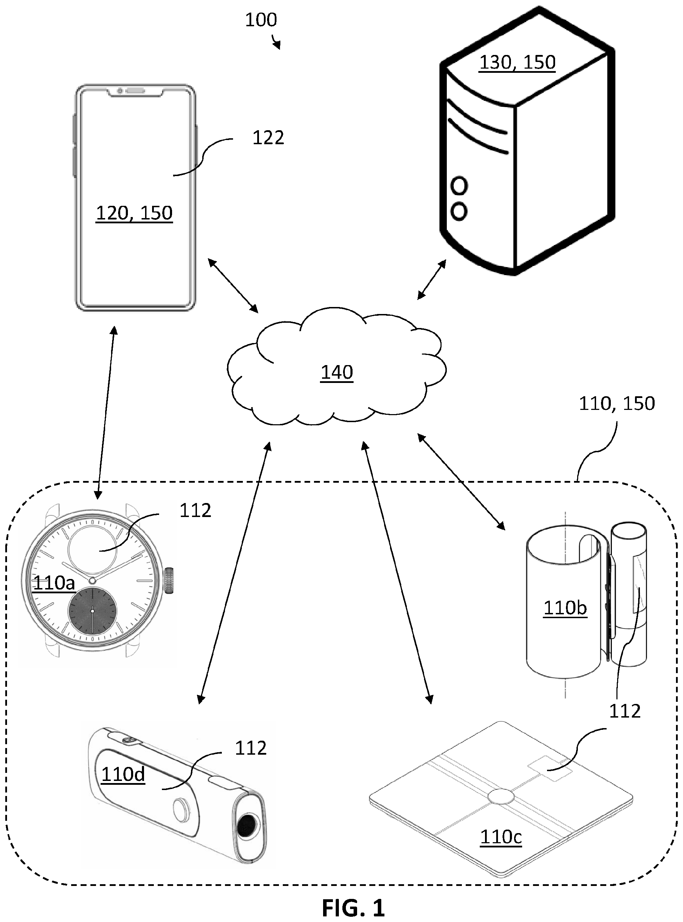

Un système de suivi 100 d'un utilisateur est illustré sur la figure 1. Le système de suivi 100 comprend un ou plusieurs dispositifs de mesure physiologique 110, un terminal mobile 120 et/ou un serveur 130, ainsi qu'un réseau de communication 140.

### Dispositif de mesure physiologique

Chaque dispositif de mesure physiologique 110 est configuré pour mesurer au moins un signal physiologique de l'utilisateur, notamment via au moins un ou plusieurs capteurs, afin de générer un ou des données de paramètre physiologique de l'utilisateur. Un paramètre physiologique est un paramètre lié directement ou indirectement au fonctionnement du corps de l'utilisateur. En particulier, le système de suivi 100, voire chaque dispositif de mesure physiologique 110, est configuré pour générer une donnée d'un des paramètres physiologiques et pour associer à la donnée un horodatage représentatif du moment où la mesure de la donnée est réalisée.

Comme illustré sur la figure 1, le ou les dispositifs de mesure physiologique 110 peuvent être, par exemple, une montre 110a, un tensiomètre 110b, un pèse-personne 110c, un multiscope 110d (tel que divulgué dans EP24217550 ou des dispositifs appelés BeamO^{™}), mais pourrait comprendre d'autres dispositifs, tels que par exemple ceux divulgués dans EP4386383 à Withings^{™}, ou des dispositifs U-Scan^{™} développés par Withings^{™}.

Chaque paramètre physiologique mesuré est notamment choisi parmi : une température du poignet, une température corporelle, une fréquence cardiaque nocturne, une fréquence cardiaque au repos, une saturation en oxygène du sang (SpO2), un indice d'apnée-hypopnée (AHI), un poids ou une masse, une masse graisseuse (en poids ou en pourcentage), un masse musculaire (en poids ou en pourcentage), une vitesse de l'onde de pouls (VOP, ou PWV), une variabilité de la fréquence cardiaque (VFC, ou HRV), un nombre de pas quotidien, une activité sportive (durée, intensité, etc.), une durée du sommeil, un score de qualité de sommeil, une heure du coucher, une heure du lever, la fréquence respiratoire, la pression ou tension artérielle, un niveau d'un composé présent dans l'urine (niveau hormonal, niveau de métabolite, etc.). Cette liste n'est pas limitative et dépend notamment des métriques accessibles par le dispositif de mesure 110.

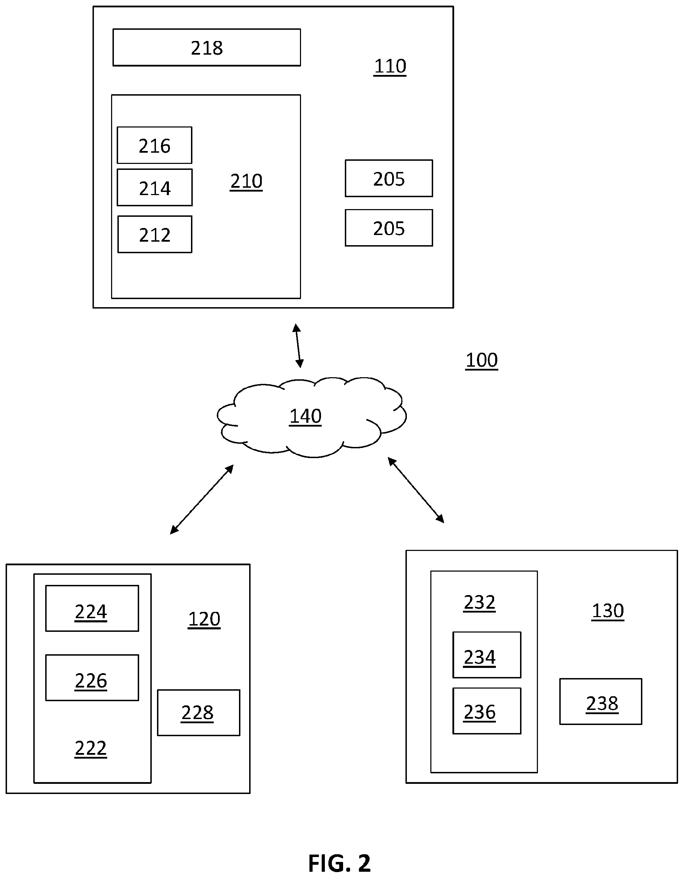

En référence à la figure 2, chaque dispositif de mesure physiologique 110 comprend une circuiterie de contrôle 210 avec un processeur 212 et une mémoire 214, et une interface entrée/sortie 216 (« input/output », I/O), qui permet notamment à la circuiterie de contrôle de recevoir et d'envoyer des données au terminal mobile 120 ou au réseau de communication 140. Le processeur 212 est configuré pour notamment traiter des données obtenues par le ou les capteurs 205 du dispositif de mesure 110. En particulier, le processeur 212 peut exécuter des instructions d'un programme stocké dans la mémoire 214. La circuiterie de contrôle 210 peut comprendre un microcontrôleur, qui intègre le processeur 212, la mémoire 214 et l'interface entrée/sortie 216. Le dispositif de mesure physiologique 110 comprend une batterie 218, apte à alimenter en énergie les différents composants du dispositif de mesure 110.

Chaque dispositif de mesure physiologique 110 peut comprendre en outre une interface d'utilisateur 112 (« user interface », UI) configurée pour afficher des informations à l'utilisateur et lui permettre, le cas échant, d'entrer des informations. Le terminal mobile 120 est en outre configuré pour afficher des informations à l'utilisateur via l'interface d'utilisateur 112, et notamment les données du ou des paramètres physiologiques.

### Terminal mobile

Le terminal mobile 120 est par exemple un smartphone (anglicisme courant, ou téléphone intelligent en français), une tablette numérique ou un ordinateur personnel.

Le terminal mobile 120 peut comprendre une circuiterie de contrôle 222, incluant un processeur 224 et une mémoire 226, et une interface entrée/sortie (« input/output », I/O) 228, qui permet à la circuiterie de contrôle de recevoir et d'envoyer des données.

En particulier, le terminal mobile 120 est configuré pour échanger des données à distance (sans fil) de manière bidirectionnelle avec l'un des dispositifs de mesure physiologique 110. La connexion peut se faire via Bluetooth, par exemple Bluetooth Low Energy (BLE). Le terminal mobile 120 peut notamment recevoir les données de paramètres physiologiques mesurées par le dispositif de mesure physiologique 110. Le terminal mobile 120 peut également envoyer des données au dispositif de mesure physiologique 110 (heure, données d'alarme, notifications, etc.). Dans le mode de réalisation illustré sur la figure 1, le terminal mobile 120 est connecté à la montre 110a.

Le terminal mobile 120 comprend en outre une interface d'utilisateur 122 (usuellement appelée « user interface », UI en anglais) configurée pour afficher des informations à l'utilisateur et lui permettre, le cas échant, d'entrer des informations (comme la taille, le sexe, etc.). Le terminal mobile 120 est en outre configuré pour afficher des informations à l'utilisateur via l'interface d'utilisateur 122, et notamment les données du ou des paramètres physiologiques en fonction du temps, par exemples sous formes de graphique.

La circuiterie de contrôle 222 est en outre configurée pour faire fonctionner une application gérant l'environnement du dispositif de mesure 110 et l'affichage des données de paramètre physiologique.

### Serveur

Le serveur 130 est une unité matérielle distante configurée pour recevoir, stocker et traiter les données reçues envoyés par le ou les dispositifs de mesure physiologique 110. Inversement, le serveur 130 est également configuré pour envoyer les données reçues des dispositifs de mesure physiologique 110 ou toute autre donnée connexe vers le terminal mobile 120. Le serveur 130 est généralement situé à distance des dispositifs de mesure physiologique 110 et du terminal mobile 120. Le serveur 130 peut être situé dans un centre de données (« data center » en anglais). Le serveur 130 peut stocker les comptes d'utilisateurs (avec toutes les données associées) de manière centralisée et sert de sauvegarde. Le serveur 130 peut comprendre plusieurs serveurs. Au sens de la présente description, toute combinaison de ces serveurs est considérée comme le serveur 130.

Le serveur 130 peut comprendre une circuiterie de contrôle 232, incluant un processeur 234 et une mémoire 236, et une interface entrée/sortie (« input/output », I/O) 238, qui permet à la circuiterie de contrôle de recevoir et d'envoyer des données vers le réseau de communication 140.

### Réseau de communication

Le réseau de communication 140 est notamment un réseau hybride (Ethernet, Wi-Fi, 3G, 4G, 5G, etc.). Le ou les dispositifs de mesure physiologique 110 et le serveur 130 échangent les données à l'aide du réseau de communication 140.

Dans un mode de réalisation, le dispositif de mesure physiologique 110 est configuré pour envoyer les données directement, par exemple via Wi-Fi, au serveur 130. Dans l'exemple de la figure 1, les dispositifs de mesure 110b, 110c et 110d envoient directement les données au serveur 130 via le réseau de communication 140.

En variante ou en complément, le dispositif de mesure physiologique 110 est configuré pour envoyer les données au serveur 130 via le terminal mobile 120. En particulier, le dispositif de mesure physiologique 110 est configuré pour envoyer les données au terminal mobile 120 via Bluetooth et le terminal mobile 120 est configuré pour envoyer les données au serveur 130 via Wi-Fi. Dans l'exemple de la figure 1, le dispositif de mesure 110a envoie les données au serveur 130 via le terminal mobile 120 et le réseau de communication 140.

### Dispositif électronique de génération d'alerte

Le système de suivi 100 comprend en outre au moins un dispositif de génération d'alerte 150. Le dispositif électronique de génération d'alerte 150 est configuré pour mettre en œuvre une méthode de génération d'alerte qui sera décrite plus en détail par la suite.

Dans un mode de réalisation, le dispositif de génération d'alerte 150 comprend ou est le serveur 130.

En variante ou en complément, le dispositif de génération d'alerte 150 comprend ou est le terminal mobile 120.

En variante ou en complément, le dispositif de génération d'alerte 150 comprend ou est l'un des dispositifs de mesure 110.

Le dispositif de génération d'alerte 150 comprend une circuiterie de contrôle, notamment la circuiterie de contrôle 210, 222 et/ou 232, et ainsi la circuiterie de contrôle comprend au moins un processeur et une mémoire stockant des instructions sous forme de programme apte à être exécutées par l'au moins un processeur.

Le fonctionnement du dispositif de génération d'alerte 150 sera décrit plus en détail par la suite.

### Méthode de génération d'alerte

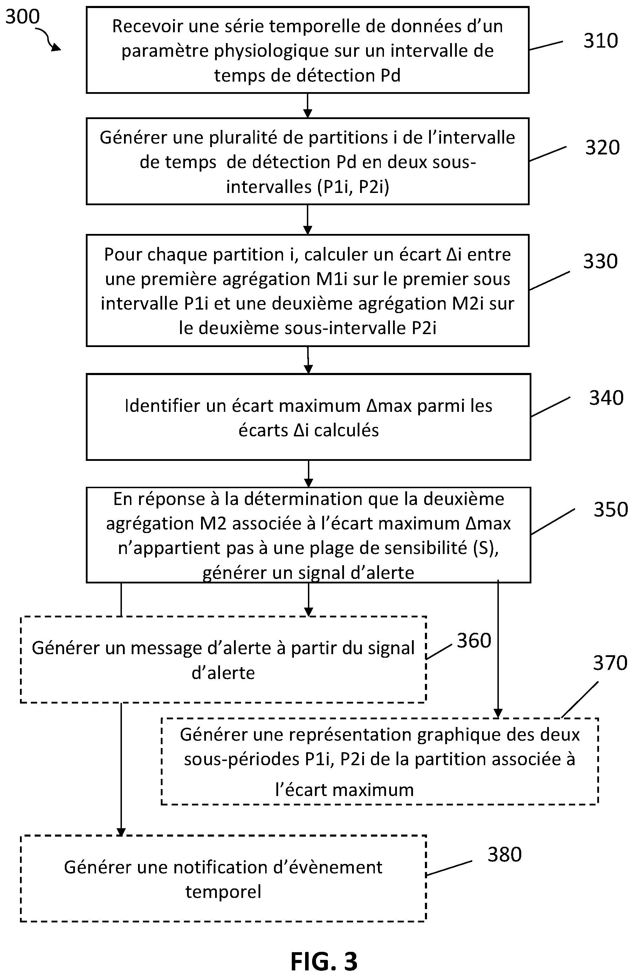

Une méthode de génération d'alerte 300 mise en œuvre par le dispositif de génération d'alerte 150 va maintenant être décrite, en référence à la figure 3.

Par souci de clarté, la méthode va être décrite dans un premier temps dans le cas d'un unique dispositif de mesure 110. La méthode 300 est mise en œuvre par la circuiterie de contrôle du dispositif de génération d'alerte 150 et notamment par le processeur qui exécute des instructions stockées dans la mémoire. Pour le reste la description, la méthode 300 sera indiquée comme étant exécutée par le dispositif de génération d'alerte 150.

Lors d'une étape initiale 310, le dispositif de génération d'alerte 150 reçoit une série temporelle de données d'un paramètre physiologique d'un utilisateur à l'intérieur d'un intervalle de temps de détection Pd et, dans un mode de réalisation, d'un intervalle de temps de sensibilité Ps. Ces deux intervalles de temps seront détaillés par la suite. Les données du paramètre physiologique seront aussi appelées données physiologiques par la suite.

Les données physiologiques sont obtenues notamment à l'aide du dispositif de mesure 110, via le ou les capteurs 205 qui mesurent un ou des signaux physiologiques. Des traitements subséquents des signaux physiologiques sont possibles, notamment via le dispositif de mesure 110, le terminal mobile 120 ou le serveur 130. Ces traitements subséquents peuvent comprendre du traitement de signal et/ou des combinaisons de signaux physiologiques.

Lorsque le dispositif de génération d'alerte 150 est le dispositif de mesure 110, les données physiologiques peuvent être reçues directement par la circuiterie de contrôle 210 qui les génèrent.

Lorsque le dispositif de génération d'alerte 150 est le serveur 130 ou le terminal mobile 120, les données physiologiques peuvent être générées puis transmises par le dispositif de mesure 110 au serveur 130 ou au terminal mobile 120 via le réseau de communication 140. Alternativement, les données physiologiques peuvent être reçues directement par le serveur 130 ou le terminal mobile 120 qui les génèrent à partir des signaux mesurés par le dispositif de mesure 110 (par exemple le serveur génère un score de sommeil qui est obtenu à partir des cycles de sommeil et des ronflements mesurés).

Comme indiqué précédemment, la série temporelle est associée à l'intervalle de temps de détection de sorte que chaque donnée de la série temporelle est associée à un horodatage se trouvant, par définition, inclus de l'intervalle de temps de détection Pd et/ou l'intervalle de sensibilité Ps. Le marqueur temporel est une indication représentative de l'instant temporel où a été réalisée la mesure ayant générée ladite donnée. L'horodatage est par exemple le jour (avec quantième, mois et année) et l'heure de la mesure.

L'intervalle de temps de détection Pd présente une durée qui est prédéfinie, c'est-à-dire que cette durée est définie préalablement à l'étape 310. En particulier, l'intervalle de temps de détection Pd présente une durée prédéfinie associée au paramètre physiologique mesuré. L'intervalle de temps de détection Pd comprend une borne de début et une borne de fin, séparées par la durée prédéfinie. L'intervalle de temps est typiquement continu. La série temporelle de données physiologiques comprend donc les données physiologiques dont l'horodatage se trouve compris dans l'intervalle de temps de détection Pd.

Dans un mode de réalisation, l'intervalle de temps de sensibilité Ps présente une durée qui est prédéfinie, c'est-à-dire que cette durée est définie préalablement à l'étape 310. En particulier, l'intervalle de temps de sensibilité Ps présente une durée prédéfinie associée au paramètre physiologique mesuré. L'intervalle de temps de sensibilité Ps comprend une borne de début et une borne de fin, séparées par la durée prédéfinie. L'intervalle de temps de sensibilité Ps est typiquement continu. La série temporelle de données physiologiques comprend donc les données physiologiques dont l'horodatage se trouve compris dans l'intervalle de temps de sensibilité Ps.

Dans un mode de réalisation, l'intervalle de temps de sensibilité Ps présente une durée plus longue que l'intervalle de temps de détection Pd. En particulier, la durée de l'intervalle de temps de sensibilité Ps est au moins 50% plus long, notamment deux fois plus long que la durée de l'intervalle de temps de détection Pd.

Dans un mode de réalisation, les deux intervalles de temps Ps, Pd présentent une intersection commune. En particulier, l'intervalle de temps de détection Pd est inclus dans l'intervalle de temps de sensibilité Ps. En variante, les deux intervalles de temps présentent chacun une plage de valeur propre.

En variante, l'intervalle de temps de sensibilité Ps est l'intervalle de temps de détection Pd.

Par exemple, la durée de l'intervalle de temps de détection Pd est de plusieurs semaines voire plusieurs mois pour le suivi du poids de l'utilisateur mais seulement de plusieurs jours pour le suivi de la température corporelle de l'utilisateur. Par exemple, pour le suivi du nombre de pas parcouru par l'utilisateur, la durée de l'intervalle de temps de détection Pd est de 30 jours et la durée de l'intervalle de temps de sensibilité Ps est de 60 jours. Chaque durée d'intervalle de temps est par exemple comprise entre 5 jours et 10 ans. Typiquement, la circuiterie de contrôle du dispositif de génération d'alerte 150 stocke une base de données associant différents intervalles de temps en fonction des paramètres physiologiques mesurés.

Chaque intervalle de temps Pd, Ps est notamment une fenêtre glissante. Autrement dit, chaque intervalle de temps est une fenêtre temporelle de durée fixe mais dont le début se déplace dans le temps, typiquement à la même vitesse que l'écoulement du temps. Par exemple, une fenêtre de 10 jours qui se termine aujourd'hui inclut les 10 jours précédent et incluant aujourd'hui, tandis qu'une fenêtre de 10 jours qui se terminait hier inclut les 10 jours précédent et incluant hier.

En particulier, l'intervalle de temps de détection Pd peut comprendre le dernier instant de réception des données physiologiques par le dispositif de génération d'alerte 150 comme borne de fin.

Dans un mode de réalisation, la borne de fin de l'intervalle de temps de sensibilité Ps est antérieur à la borne de début de l'intervalle de temps de détection Pd.

L'intervalle de temps est associé à une unité de temps. L'unité de temps définit une division temporelle régulière de l'intervalle de temps. L'unité de temps est par exemple une heure, une demi-journée, une journée, une pluralité de jours, une semaine, un mois, etc.

Dans un mode de réalisation, le dispositif de génération d'alerte 150 effectue un pré-traitement des données physiologiques sur chaque unité de temps suite à la réception des données physiologiques.

En particulier, le dispositif de génération d'alerte 150 moyenne les données physiologiques sur chaque unité de temps suite à la réception des données physiologiques.

Par exemple, des données de rythme cardiaques sont moyennées toutes les heures ou sur la nuit. La température corporelle peut être moyennée sur une journée. Le poids peut être moyenné sur une semaine.

Pour la suite de la description, la moyenne est par exemple une moyenne arithmétique, une moyenne harmonique, une moyenne géométrique, une moyenne quadratique, etc.

En variante, le dispositif de génération d'alerte 150 peut prendre le maximum ou le minimum des données physiologiques sur chaque unité de temps comme pré-traitement des données. Par exemple, le dispositif de génération d'alerte 150 peut prendre le minimum de poids sur la semaine, le minimum de rythme cardiaque pour la nuit ou le maximum de température corporelle sur la journée.

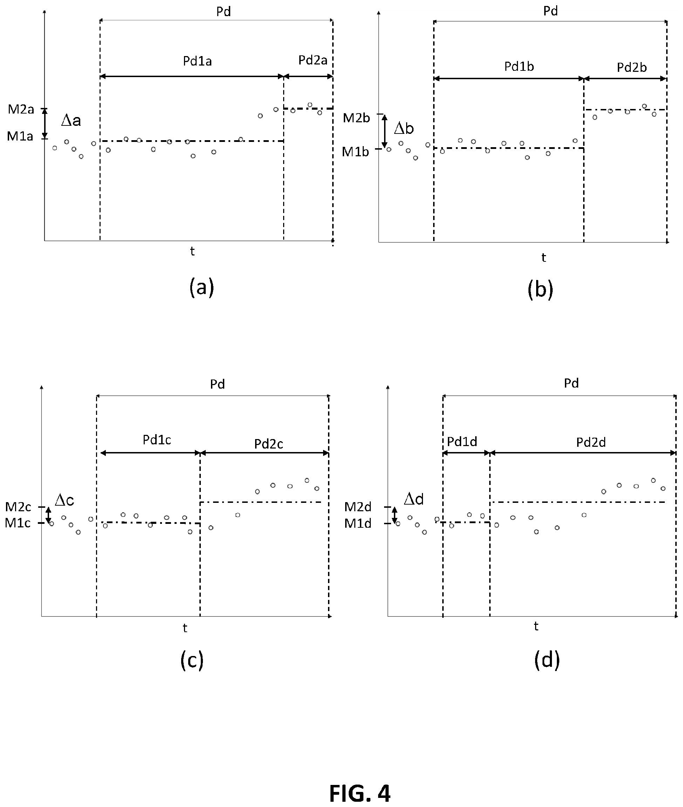

Puis lors d'une étape 320, le dispositif de génération d'alerte 150 génère une pluralité de partitions de l'intervalle de temps de détection Pd en deux sous-intervalles continus, avec un premier sous-intervalle Pd1 et un deuxième sous-intervalle Pd2. Le deuxième sous-intervalle Pd2 est définie comme temporellement succédant le premier sous-intervalle Pd1. Des exemples sont visibles en figure 4 qui sera décrite par la suite.

Une partition de l'intervalle de temps de détection est une division de l'intervalle de temps de détection en strictement deux sous-intervalles continus, les deux sous-intervalles continus couvrant l'intégralité de l'intervalle de temps. Par continu, il est signifié qu'il n'existe pas, entre les horodatages de deux données physiologiques d'un sous-intervalle, un horodatage d'une donnée physiologique de l'autre sous-intervalle. La partition est par exemple une partition en deux sous-intervalles égaux, une partition 10%-90% de l'intervalle de temps, une partition 40%-60%, etc. Comme cela sera décrit par la suite, la méthode peut calculer toutes les partitions possibles. Par conséquent, toute donnée physiologique de l'intervalle de temps de détection se trouve exactement et uniquement dans l'un ou l'autre des sous-intervalles de temps.

Dans un mode de réalisation, un critère de durée minimale prédéfinie des sous-intervalles est appliqué. Ainsi, tout sous-intervalle de toute partition possible présente une durée minimale. Cela permet d'assurer que le sous-intervalle est suffisamment long pour être représentatif et ne pas être sensible aux erreurs de mesure ou valeurs aberrantes.

La durée minimale prédéfinie de chaque sous-intervalle peut être fonction de la durée prédéfinie de l'intervalle de temps de détection. Par exemple, chaque sous-intervalle présente une durée au moins égale à un cinquième de la durée de l'intervalle de temps de détection total.

En variante ou en complément, la durée minimale prédéfinie peut être associée à un nombre minimal de données, indépendamment d'une durée temporelle qui comprend les dates de ces données physiologiques. Par exemple, chaque sous-intervalle comprend au moins deux, voire trois, données physiologiques.

En variante ou en complément, la durée minimale prédéfinie de chaque sous-intervalle est fonction du paramètre physiologique. Par exemple, chaque sous-intervalle de temps dure au moins 2 jours pour la température corporelle, plusieurs jours pour les pas, une semaine pour le poids.

La figure 4 illustre quatre partitions différentes (a, b, c, d) d'un intervalle de temps de détection P, telles que générées à l'étape 320. Le reste de la description utilisera le terme de partition i, avec i allant de a à d dans l'exemple de la figure 4. L'intervalle de temps de détection Pd est ainsi divisé en quatre paires différentes de sous-intervalles (Pd1i, Pd2i, avec i allant de a à d).

Dans un mode de réalisation, le dispositif de génération d'alerte 150 est configuré pour définir toutes les partitions possibles selon l'unité de temps de l'intervalle de temps de détection Pd en deux sous-intervalles continus, avec , lorsque appliquée, la condition supplémentaire que chaque sous-intervalle présente la durée minimale prédéfinie. Par exemple, si la durée prédéfinie de l'intervalle de temps de détection est une semaine, que l'unité de mesure associée est la journée et que la durée minimale prédéfinie pour les sous-intervalles est de 2 jours, le dispositif de génération d'alerte 150 génère les partitions suivantes : [2 premiers jours - 5 derniers jours], [3 premiers jours - 4 derniers jours], [4 premiers jours - 3 derniers jours] et [5 premiers jours - 2 derniers jours].

Puis lors d'une étape 330, le dispositif de génération d'alerte 150 calcule, pour chaque partition i générée à l'étape 320, un écart Δi entre un résultat d'une fonction d'agrégation appliquée sur des données dont l'horodatage est inclus dans le premier sous-intervalle P1i, appelée première agrégation M1i, et un résultat de la fonction d'agrégation appliquée sur des données physiologiques dont l'horodatage est inclus dans le deuxième sous-intervalle P2i, appelée deuxième agrégation M2i. La première agrégation M1i et la deuxième agrégation M2i sont chacune un nombre unique, de type scalaire, c'est-à-dire comparable selon une relation d'ordre. La fonction d'agrégation appliquée au premier sous-intervalle est la même que celle appliquée au deuxième sous-intervalle.

La fonction d'agrégation est un opérateur permettant de réduire un ensemble de données physiologiques à une seule valeur. La fonction d'agrégation est choisie notamment parmi : une moyenne, une médiane, un écart-type, un maximum, un minimum.

Lorsque les données physiologiques ont été pré-traitées selon l'unité de temps, la première agrégation M1i et la deuxième agrégation M2i sont réalisées à partir des données pré-traitées par unité de temps.

En particulier, l'écart Δi entre les deux agrégations M1i et M2i est la valeur absolue de la différence entre ces deux agrégations. Ainsi Δi=|M1i-M2i|.

Sur l'exemple de la figure 4, la fonction d'agrégation est une moyenne. Les moyennes M1i et M2i ont été calculées et représentées pour chaque sous-intervalle P1i, P2i de chaque partition i. L'écart Δi entre M1i et M2i est également représenté pour chaque partition i. On remarque ainsi qu'en fonction des partitions i, la répartition des données physiologiques entre les deux sous-intervalles changent, ce qui fait aussi changer les moyennes des sous-intervalles. Dans les exemples de la figure 4, on remarque que les moyennes Δc, Δd sont nettement inférieures aux moyennes Δa, Δb. Au vu en outre de la réparation des données entre les partitions a et b, on remarque que la moyenne Δb est supérieure à la moyenne Δa.

Puis lors d'une étape 340, le dispositif de génération d'alerte 150 identifie un écart maximum Δmax parmi les écarts Δi calculés. Autrement dit : Δmax = max (Δi).

Sur l'exemple de la figure 4, les différents écarts Δa, Δb, Δc, Δd sont comparés entre eux. On remarque que les écarts Δc et Δd sont les plus faibles parmi les écarts et qu'ainsi les deux partitions (P1c, P2c) et (P1d, P2d) ne conduisent pas à des changements de tendance importants. Les écarts Δa et Δb sont plus importants et sont donc associés à des partitions conduisant à des changements de tendance plus important entre les deux sous-intervalles. L'écart maximum est celui du cas b. Le module de génération d'alerte identifie donc que l'écart maximum Δmax est celui de la partition b). Ainsi dans cet exemple Δmax = Δb.

Puis lors d'une étape 350, en réponse à la détermination que la deuxième agrégation M2 associée à l'écart maximum Δmax n'appartient pas à une plage de sensibilité S, le dispositif de génération d'alerte 150 génère un signal d'alerte.

Le signal d'alerte peut comprendre une information relative à au moins l'un des éléments suivants : la partition (P1i, P2i) associé à l'écart maximum Δmax, la première agrégation M1i et la deuxième agrégation M2i associées et/ou l'écart maximum Δmax.

La plage de sensibilité S est établie de sorte que la deuxième agrégation M2 présente un changement de tendance jugé significatif et que la plage de sensibilité S conduise à une génération d'un signal d'alerte pertinent.

Dans un mode de réalisation, la plage de sensibilité S est fonction de l'historique des données du paramètre physiologique sur l'intervalle de temps de sensibilité Ps. En particulier, la plage de sensibilité S est un intervalle de valeurs centré sur une référence Ref. La référence Ref est notamment le résultat de ladite fonction d'agrégation appliquée sur des données associées à l'intervalle de temps de sensibilité Ps. Autrement dit, la fonction d'agrégation utilisée à l'étape 330 est la même que celle utilisée pour calculer la référence Ref. L'agrégation sur l'intervalle de temps de sensibilité Ps représente la valeur de référence du paramètre physiologique sur l'intervalle de temps de sensibilité Ps, dont la longueur est notamment plus grande que la longueur de l'intervalle de temps de détection Pd. Cela permet de comparer la deuxième agrégation M2 à une référence Ref calculée sur une période plus grande et dont la valeur est fiable.

Dans un mode de réalisation, l'intervalle de temps de sensibilité Ps et le deuxième sous-intervalle Pd2 sont disjoints, ce qui signifie qu'il n'existe pas de données physiologiques dont l'horodatage est inclus dans le deuxième sous-intervalle Pd2 et dans l'intervalle de temps de sensibilité Ps. En particulier, l'intervalle de temps de sensibilité comprend le premier sous-intervalle Pd1. Comme expliqué plus haut, l'intervalle de temps de sensibilité Ps peut toutefois s'étendre au-delà du premier sous-intervalle Pd1 dans le passé pour calculer la référence sur une période plus longue. Ainsi, dans ce mode de réalisation qui exclut le deuxième sous-intervalle, le calcul de la référence Ref n'est pas perturbé par l'éventuel changement de tendance des données physiologiques.

La plage de sensibilité est égale à la référence Ref plus ou moins un seuil Th prédéterminé. Autrement dit, lors de l'étape 350, le dispositif de génération d'alerte 150 génère un signal d'alerte si l'écart entre la deuxième moyenne M2 et la référence Ref est supérieure au seuil Th.

A cet effet, le seuil Th prédéterminé peut être fonction du paramètre physiologique. Par exemple, le seuil Th prédéterminé relatif à un suivi d'un nombre de pas parcourus par jour sera plus grand en relatif par rapport à la moyenne habituelle du nombre de pas que le seuil relatif à la température corporelle car les pas parcourus chaque jour sont souvent plus variables que la température corporelle d'un jour à l'autre. Il est ainsi défini pour chaque paramètre physiologique, un seuil associé. Chaque seuil est par exemple défini préalablement par des experts médicaux. Ce seuil est défini en absolu ou en relatif, comme cela sera décrit ci-dessous.

En variante ou en complément, le seuil Th prédéterminé est fonction de l'historique des données physiologiques. Le seuil est ainsi défini en relatif par rapport à l'historique des données physiologiques sur l'intervalle de temps de sensibilité Ps. En particulier, le seuil peut être défini en fonction de l'écart-type sur l'intervalle de temps de sensibilité Ps. Par exemple, si l'intervalle de temps de détection Pd est d'une semaine, le seuil Th peut être défini comme deux fois l'écart type sur l'intervalle de temps de sensibilité Ps égal à 60 derniers jours. En variante ou en complément, le seuil Th peut être défini en fonction de la moyenne sur l'intervalle de temps de sensibilité Ps. Par exemple, le seuil Th peut être défini comme 20% de la moyenne sur l'intervalle de temps de sensibilité Ps égal sur les 60 derniers jours.

Dans un mode de réalisation, le seuil Th peut être fonction du paramètre physiologique et de l'historique des données physiologiques de l'utilisateur.

En variante ou en complément, le seuil Th peut être fonction d'un niveau de sensibilité prédéfini en fonction du paramètre physiologique, par exemple par l'utilisateur ou un professionnel de santé. Le niveau de sensibilité est un paramètre, par exemple à choisir parmi « bas », « moyen » et « haut », définissant la sensibilité souhaitée de l'alerte, autrement dit à quel point l'utilisateur ou le professionnel de santé souhaite être informé rapidement d'un changement de tendance. Plus le niveau de sensibilité défini est élevé, moins le seuil Th prédéterminé est élevé et plus un signal d'alerte peut être envoyé facilement, avec éventuellement un risque de faux positif. A l'inverse, un niveau de sensibilité faible est associé avec un seuil élevé ce qui limite la génération de signal d'alerte.

Dans un mode de réalisation, le dispositif de génération d'alerte 150 génère le signal d'alerte à la condition supplémentaire que le premier sous-intervalle Pd1 soit plus grand (e.g., plus long en matière temporelle ou avec plus de données) que le deuxième sous-intervalle. Alternativement, cette condition se traduit au niveau des partitions générées à l'époque 320 (indépendamment des autres critères déjà présentés) : le dispositif de génération d'alerte 150 ne génère que des partitions dont le premier sous-intervalle est plus grand ou long que le deuxième sous-intervalle.

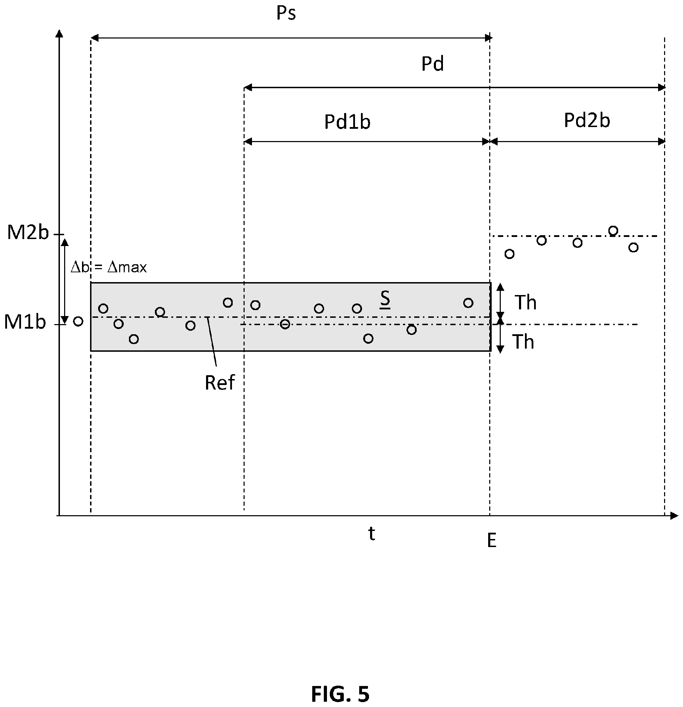

Pour continuer l'exemple de la figure 4, la figure 5 reprend la partition b) de la figure 4 qui a été identifiée lors de l'étape 340 comme étant associée à l'écart maximum Δmax. La plage de sensibilité S définie par plus ou moins le seuil Th autour de la référence Ref est représentée sur la figure 5. Dans cet exemple, la deuxième agrégation M2b est hors de la plage de sensibilité S. Le dispositif de génération d'alerte 150 génère alors un signal d'alerte.

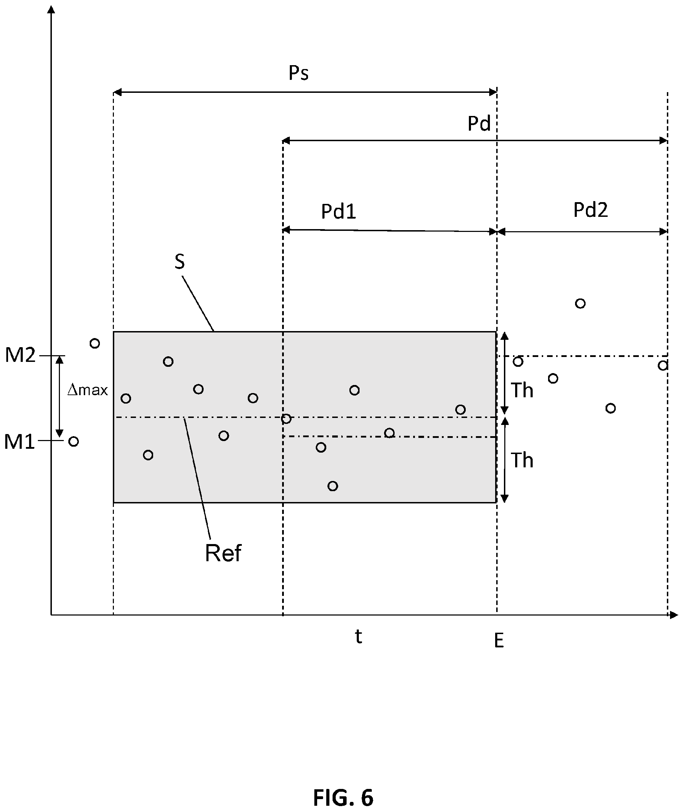

La figure 6 représente un autre exemple avec une série temporelle de données physiologiques avec une variabilité au cours du temps plus importante que celle des données de la figure 4. En ordonnées est représenté le nombre de pas parcourus par jour, qui peut varier du simple au double d'un jour à l'autre et en abscisses le temps. Dans cet exemple, la deuxième agrégation M2b se situe dans la plage de sensibilité S. Le seuil Th est ici assez large du fait de la variabilité de l'historique des données physiologiques. Le dispositif de génération 150 estime dans ce cas que la variation observée entre la deuxième moyenne M2 et la référence Ref n'est pas significative pour conduire à la génération d'un signal d'alerte pertinent.

Lors d'une étape 360 optionnelle, le dispositif de génération d'alerte 150 génère un message d'alerte à partir du signal d'alerte. Le message d'alerte est notamment destiné à l'utilisateur et/ou à un professionnel de santé. Dans un mode de réalisation, le dispositif de génération d'alerte 150 est configuré pour mettre en œuvre un grand modèle linguistique (ou usuellement appelée « LLM » pour « *Large Language Model* » en anglais) pour générer le message d'alerte. Le dispositif de génération d'alerte 150 envoie au moins en entrée du LLM la partition i, avec les sous-intervalles (P1i, P2i), ayant conduit à l'écart maximum et les première et deuxième agrégations M1i, M2i associées aux sous-intervalles. Le message d'alerte est par exemple un message expliquant la différence de tendance détectée et les conséquences potentielles sur le mode de vie de l'utilisateur, et/ou des conseils et astuces pour améliorer son mode de vie. Le LLM peut également prendre en entrée des données de contexte pour rendre le message d'alerte plus pertinent, comme par exemple un historique des données physiologiques de l'utilisateur ou les valeurs d'autres données physiologiques pendant l'intervalle de temps de détection Pd.

Le dispositif de génération d'alerte 150 envoie le message d'alerte au terminal mobile 120 et/ou au dispositif de mesure 110 et/ou à un terminal externe de suivi de patient pour affichage du message d'alerte à l'utilisateur et/ou au personnel de santé.

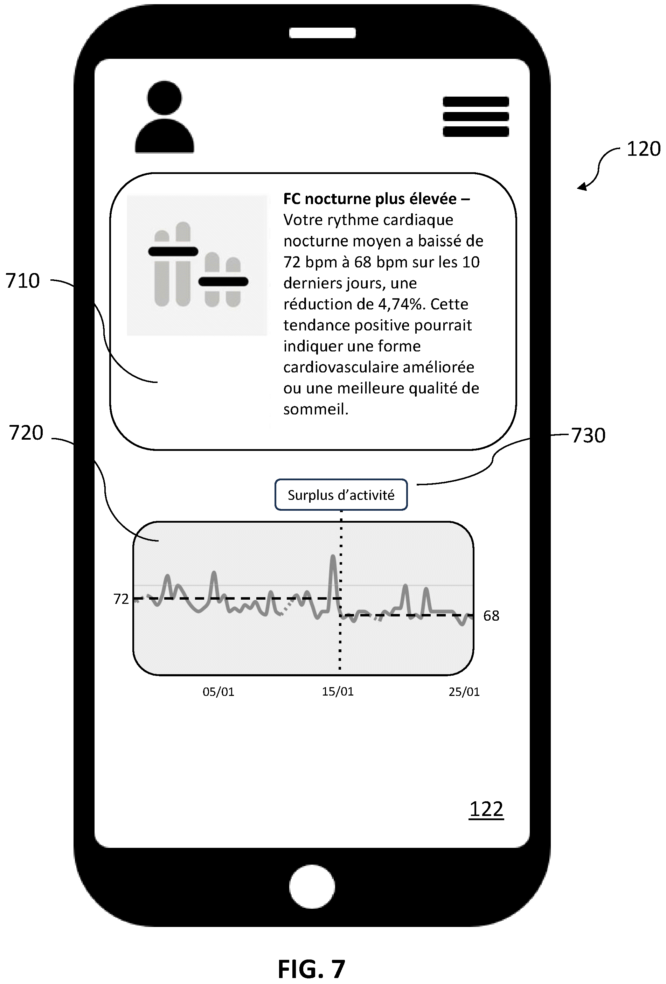

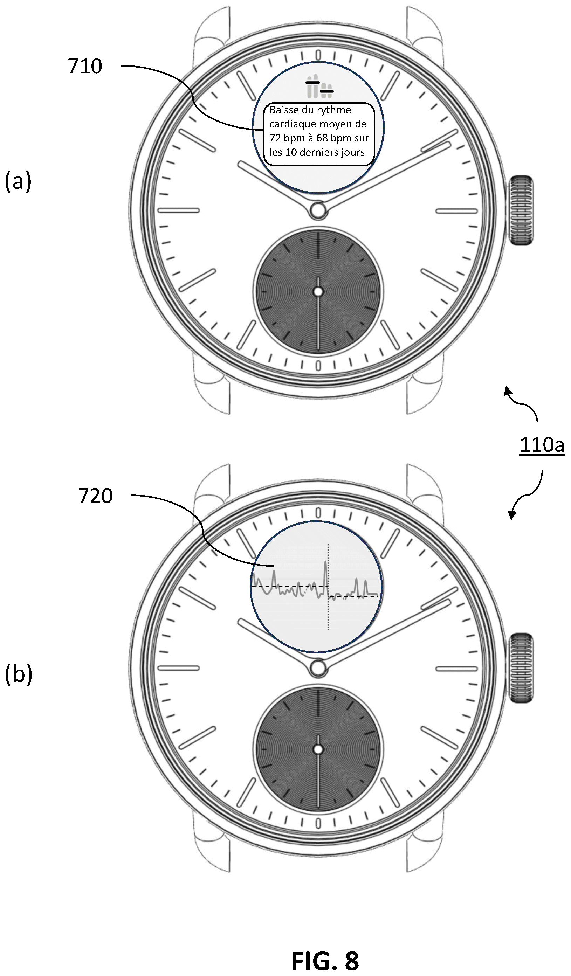

Un exemple de message d'alerte 710 affiché sur le terminal mobile 120 de l'utilisateur est illustré sur la figure 7. Le message d'alerte 710 est ici affiché dans une application dédiée mais peut être également affiché via une messagerie ou via une notification. La figure 8 illustre l'affichage de ce message d'alerte 710 sur l'écran d'une montre 110a.

Lors d'une étape 370 optionnelle, le dispositif de génération d'alerte 150 est configuré pour générer une représentation graphique représentative des deux sous-intervalles P1i, P2i de la partition associée à l'écart maximum Δmax suite à la génération d'un signal d'alerte. La représentation graphique est par exemple une représentation temporelle des données physiologiques en fonction du temps sur laquelle sont représenté les deux sous-intervalles P1i, P2i, par exemple au moyen de couleurs différentes ou d'une séparation affichée entre les deux sous-intervalles P1i, P2i.

Le dispositif de génération d'alerte 150 envoie la représentation graphique au terminal mobile 120 et/ou au dispositif de mesure 110 et/ou à un terminal externe de suivi de patient pour affichage de la représentation graphique à l'utilisateur et/ou au personnel de santé.

En variante, la représentation graphique est générée directement par le terminal mobile 120 et/ou au dispositif de mesure 110 à partir des données physiologiques et du signal d'alerte.

Un exemple de représentation graphique 720 affichée sur le terminal mobile 120 de l'utilisateur est illustré sur la figure 7. La représentation graphique 720 est ici affichée dans une application dédiée mais peut être également affiché via une messagerie ou via une notification. La figure 8 illustre l'affichage de cette représentation graphique 720 sur l'écran d'une montre 110b.

Lors d'une étape 380 optionnelle, le dispositif de génération d'alerte 150 extrait de la partition i associée à l'écart maximum Δmax un marqueur temporel diviseur E qui partitionne l'intervalle de temps de détection Pd en les deux sous-intervalles Pd1, Pd2. Autrement dit, le marqueur temporel diviseur E est le moment temporel entre les deux sous-intervalles Pd1, Pd2. Ce marqueur temporel diviseur E est illustré sur les figures 5 et 6 par exemple.

Puis, le dispositif de génération d'alerte 150 génère une notification d'évènement temporel. L'évènement temporel est associé au marqueur temporel diviseur E extrait. En particulier, le dispositif de génération d'alerte 150 détecte et associe un événement de la vie de l'utilisateur au marqueur temporel diviseur E qui marque le changement de tendance dans la série temporelle des données physiologiques.

En particulier, l'événement peut être détecté et identifié par le dispositif de génération d'alerte 150 par la mise en œuvre un modèle d'apprentissage artificiel avec au moins la partition ayant donné l'écart maximum et les fonctions d'agrégation associées en entrées.

En variante, le dispositif de génération d'alerte 150 peut utiliser une classification prédéterminée, réalisée par exemple par des experts, associant une partition et des moyennes associées à un évènement.

L'évènement est par exemple une maladie, un événement sportif, une grossesse, un événement festif, des vacances, un changement d'habitude de vie, etc.

Le dispositif de génération d'alerte 150 envoie la notification d'évènement temporel au terminal mobile 120 et/ou au dispositif de mesure 110 et/ou à un terminal externe de suivi de patient pour affichage de la notification d'évènement temporel à l'utilisateur et/ou au personnel de santé.

Un exemple de notification d'évènement temporel 730 affichée sur le terminal mobile 120 de l'utilisateur est illustré sur la figure 7. La notification d'évènement temporel 730 est ici affichée en lien avec la représentation graphique 720.

A titre d'exemple, si la fonction d'agrégation est la moyenne et si une augmentation significative de la VFC est détectée par à la référence Ref, un début de maladie ou un surentrainement est suspecté et un message d'alerte en ce sens est envoyé à l'utilisateur. Le message peut également inviter l'utilisateur à consulter un médecin.

A titre d'autre exemple, si la fonction d'agrégation est l'écart-type et si des plus grandes variations de poids sont détectées sur la dernière année par rapport à la référence Ref, même si la moyenne reste sensiblement similaire à la référence Ref, des tentatives répétées de régimes alimentaires sont suspectées et un message d'alerte en ce sens est envoyé à l'utilisateur. Le message peut également comprendre des conseils nutritionnels.

A titre d'autre exemple, si la fonction d'agrégation est le minimum et si le rythme cardiaque nocturne minimum sur le dernier mois est inférieur à la référence Ref, une augmentation de l'activité physique est suspectée et un message d'alerte en ce sens est envoyée à l'utilisateur. Le message peut également comprendre des encouragements à continuer et des exemples d'autres activités physiques à réaliser.

### Mise en œuvre répétée de la méthode

Dans un mode de réalisation, le dispositif de génération d'alerte 150 est configuré pour mettre en œuvre la méthode de génération d'alerte de manière répétée dans le temps. En particulier, le dispositif de génération d'alerte 150 est configuré pour mettre en œuvre la méthode de génération d'alerte à intervalle de temps régulier. Cet intervalle de temps régulier peut dépendre en particulier de l'unité de temps associée au paramètre physiologique. En particulier, la méthode de génération d'alerte peut être mise en œuvre à chaque unité de temps. Par exemple, si le paramètre physiologique est le poids et que l'unité de temps est la journée, la méthode de génération d'alerte est mise en œuvre tous les jours.

En complément ou en variante, le dispositif de génération d'alerte 150 est configuré pour œuvre la méthode de génération d'alerte à chaque nouvelle réception de données physiologiques, l'horodatage de la dernière donnée physiologique étant la borne de fin de l'intervalle de temps défini. Par exemple, la méthode de génération d'alerte est mise en œuvre après chaque pesée de l'utilisateur.

Lorsque la méthode de génération d'alerte est mise en œuvre à nouveau, un nouvel intervalle de temps de détection Pd et un nouvel intervalle de temps de sensibilité Ps sont définis ainsi qu'une nouvelle partition. On rappelle ici que les caractéristiques de chaque intervalle de temps, comme sa durée ou sa durée minimale sont prédéterminées. Lorsque la nouvelle mise en œuvre de la méthode est effectuée de manière rapprochée de la mise en œuvre précédente, la nouvelle partition sélectionnée suite à la mise en œuvre de la méthode (à l'étape 320 en l'occurrence) est usuellement associée au même marqueur temporel diviseur E. En effet, l'invention permet de sélectionner la partition associée à un événement de la vie de l'utilisateur comme décrit ci-dessus, cet évènement caractérisant un changement de tendance dans les données physiologiques. Tant que cet événement est compris dans l'intervalle de temps, et sous réserve d'absence d'un nouveau changement de tendance, la méthode permet de détecter cet événement et de sélectionner la partition associée. Cela sera illustré plus en détail par la suite.

Lorsqu'un signal d'alerte a été généré, le dispositif de génération d'alerte 150 peut être configuré pour générer une nouvelle alerte en réponse à la détermination que, en outre, la durée entre la génération de la précédente alerte et un horodatage associé à la dernière donnée reçue est supérieure à une durée minimale prédéterminée. Ainsi, l'utilisateur ne reçoit pas des alertes trop régulièrement. Par exemple, si l'utilisateur a reçu une alerte relative à une augmentation de sa température corporelle, une nouvelle alerte ne sera générée le lendemain mais par exemple uniquement sept jours après pour permettre d'évaluer l'évolution de la tendance et ne pas trop stresser l'utilisateur.

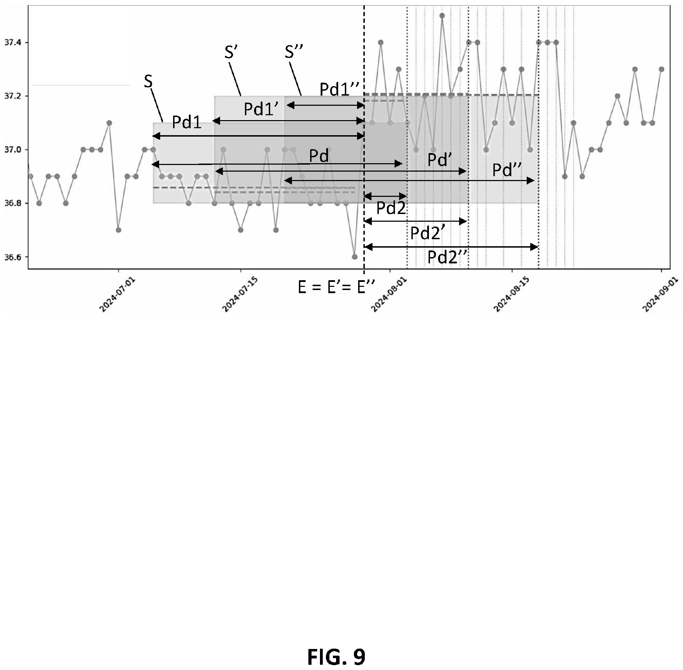

La Figure 9 représente l'évolution de la température au poignet d'un utilisateur avec comme unité de temps la journée. Par soucis de simplicité et de clarté, l'intervalle de temps de détection Pd est dans cet exemple égal à l'intervalle de temps de sensibilité Ps, soit Pd=Ps, et sera appelé intervalle de temps dans cet exemple. Trois mises en œuvre successives de la méthode de génération d'alerte sont illustrées, respectivement associées aux ensemble (Pd, Pd1, Pd2, S, E) ; (Pd', Pd1', Pd2', S', E') et (Pd", Pd1", Pd2", S", E"). La durée de l'intervalle est identique et prédéfinie pour chaque mise en œuvre, autrement dit Pd=Pd'=Pd". La première mise en œuvre de la méthode réalisée le 3 août 2021 permet de détecter un changement de tendance jugé significatif et ainsi conduit à la génération d'une alerte. Une suspicion de maladie est estimée à partir du marqueur temporel diviseur E, autour du 29 juillet 2024.

Une deuxième mise en œuvre de la méthode est réalisée une semaine après, soit le 10 août 2024. L'intervalle de temps Pd' est donc décalé d'une semaine par rapport à l'intervalle de temps Pd. Une nouvelle partition est déterminée et conduit au même marqueur temporel diviseur E'=E, ce qui confirme la suspicion de maladie autour du 29 juillet 2024. Une nouvelle alerte est générée à l'utilisateur pour le notifier que sa température au poignet est toujours élevée par rapport à sa tendance habituelle.

Une troisième mise en œuvre de la méthode est réalisée une semaine supplémentaire après, soit le 17 août 2024. L'intervalle de temps Pd" est donc décalé de deux semaines par rapport à l'intervalle de temps Pd. Une nouvelle partition est déterminée et qui conduit au même marqueur temporel diviseur E"=E'=E, ce qui confirme encore la suspicion de maladie autour du 29 juillet 2024. Une nouvelle alerte est générée à l'utilisateur pour le notifier que sa température au poignet est toujours élevée par rapport à sa tendance habituelle.

Cet exemple démontre l'intérêt de la méthode selon l'invention qui permet de définir plusieurs partitions successives au cours du temps associées un même marqueur temporel diviseur, ce marqueur étant lié à un événement réel de la vie de l'utilisateur, et non une simple comparaison de moyennes sur des périodes arbitraires comme suggéré dans l'état de l'art.

### Mode de réalisation avec plusieurs dispositifs de mesure

Lorsque le système de suivi 100 comprend une pluralité de dispositifs de mesure 110, le dispositif de génération d'alerte 150 est configuré pour mettre en œuvre la méthode de génération d'alerte pour chaque paramètre physiologique mesuré.

Lorsque plusieurs paramètres physiologiques sont suivis par l'utilisateur, il peut être défini un paramètre physiologique dit principal et un ou plusieurs paramètres physiologiques dit secondaires. Le paramètre physiologique principal est par exemple le paramètre physiologique que l'utilisateur ou un professionnel de santé a défini comme étant le plus important à suivre. Par exemple, si un utilisateur veut suivre en priorité son poids, le poids est le paramètre physiologique principal et le nombre de pas parcourus chaque jour est un paramètre physiologique secondaire.

Dans un mode de réalisation, le dispositif de génération d'alerte 150 définit le même intervalle de temps de détection Pd et l'intervalle de temps de sensibilité Ps pour chaque paramètre physiologique. En particulier, chaque intervalle de temps peut être choisi sur la base du paramètre physiologique principal. La mise en œuvre de chaque méthode de génération d'alerte peut aboutir à des partitions différentes. Ainsi, l'invention permet de détecter sur un même intervalle de temps de détection Pd à quel moment les données des différents paramètres physiologiques suivis changent ou non de tendance. Par exemple, si l'utilisateur suit principalement son poids et qu'une prise de poids a été détecté dans les derniers jours, l'analyse sur le même intervalle de temps de détection du nombre de pas parcourus peut montrer une baisse de ces pas parcourus depuis plus de deux semaines, ce qui peut expliquer en partie cette prise de poids.

En variante, la même partition est utilisée pour tous les paramètres physiologiques. En particulier, la partition est sélectionnée sur la base de l'analyse du paramètre physiologique principal. Sur la base de cette même partition, le dispositif de génération d'alerte 150 est configuré pour calculer l'écart entre une première moyenne des données d'un paramètre physiologique secondaire associées au premier sous-intervalle avec une deuxième moyenne des données du paramètre physiologique secondaire associées au deuxième sous-intervalle, et pour générer un signal d'alerte si l'écart est supérieur à un seuil prédéterminé. Ainsi, la méthode permet d'analyser si des paramètres physiologiques secondaires présentent la même variation de tendance que le paramètre physiologique principal sur la même partition, et ainsi affiner la détection de l'événement associé à ces variations. En particulier, le dispositif de génération d'alerte 150 est configuré pour mettre en œuvre le modèle d'apprentissage artificiel avec au moins la partition et les moyennes associées aux différents paramètres physiologiques en entrées pour détecter un événement pendant l'intervalle de temps.

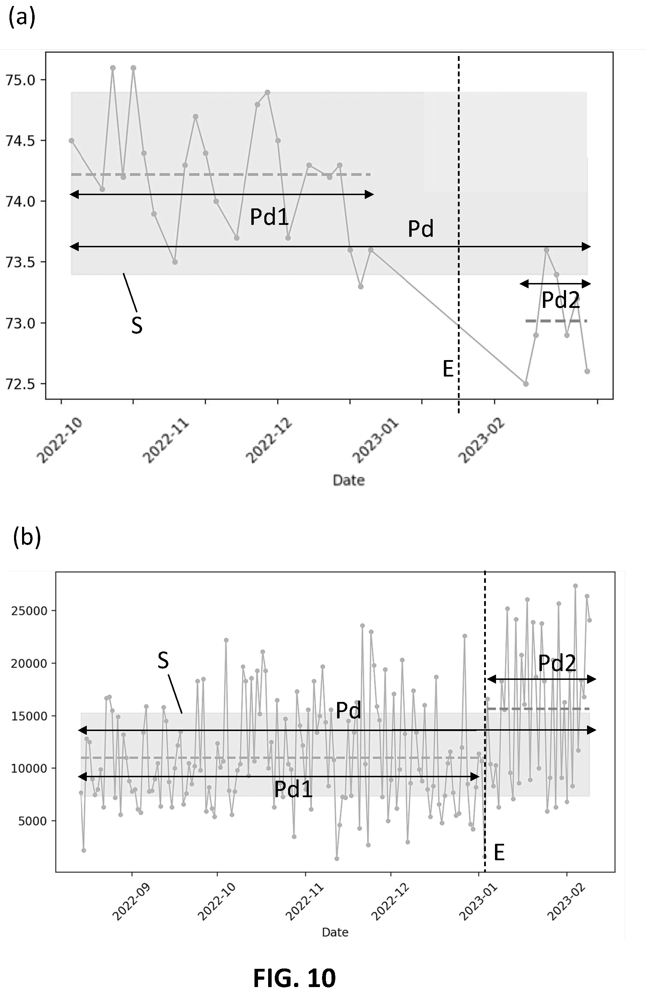

A titre exemple, si une perte de poids est détectée sur un intervalle de temps et si le nombre de pas augmente, alors un changement d'habitude de vie est suspectée, par exemple une activité sportive renforcée. Ce cas est illustré sur la Figure 10. Dans cet exemple, l'intervalle de temps de détection Pd est égal à l'intervalle de temps de sensibilité Ps, appelé intervalle de temps. Chaque paramètre physiologique est associé à son propre intervalle de temps et sa propre partition. Une baisse de poids significative est détectée autour de janvier 2023 pour un utilisateur. Cette baisse de poids est corrélée avec une augmentation significative du nombre de pas à partir de janvier 2023 pour cet utilisateur, suggérant par exemple des aller-retours au travail en marchant ou l'augmentation du nombre de séances de sport dans la semaine. A l'inverse de cet exemple, si le nombre de pas baisse, une maladie de l'utilisateur est suspectée.

A titre d'autre exemple, si une baisse de la qualité du sommeil est détectée et si le nombre de pas parcourus diminue sur le même intervalle de temps, une maladie peut être suspectée. A l'inverse, si le nombre de pas augmente, un surentrainement ou des vacances peuvent être suspectés. L'analyse de l'heure de sommeil peut permettre d'affiner ce diagnostic. En particulier, si l'heure de mise au lit est plus tardive des vacances sont détectées alors que si l'heure de mise au lit ne varie pas significativement, l'hypothèse d'un surentrainement est privilégiée.

A titre d'autre exemple, si une baisse de la VFC est détectée sur un intervalle de temps et si la température corporelle de l'utilisateur augmente sur cet intervalle de temps, une maladie est suspectée. Si la température corporelle est relativement constante, l'hypothèse d'un épisode de stress est privilégiée.

## Revendications

1. Dispositif de génération d'alerte (150) comprenant une circuiterie de contrôle configurée pour mettre en œuvre une méthode (300) de génération d'alerte, la méthode de génération d'alerte comprenant les étapes suivantes :
- recevoir (310) une série temporelle de données d'un paramètre physiologique d'un utilisateur, dites données physiologiques, les données physiologiques étant associées à un intervalle de temps de détection (Pd), chaque donnée physiologique de la série temporelle étant associée à un horodatage inclus de l'intervalle de temps de détection,
- générer (320) une pluralité de partitions de l'intervalle de temps de détection (Pd) en deux sous-intervalles (P1i, P2i) continus, avec un premier sous-intervalle (P1i) suivi d'un deuxième sous-intervalle (P2i),
- pour chaque partition générée, calculer (330) un écart (Δi) entre un résultat d'une fonction d'agrégation appliquée sur des données physiologiques dont l'horodatage est inclus dans le premier sous-intervalle, appelée première agrégation (M1i), et un résultat de la fonction d'agrégation appliquée sur des données dont l'horodatage est inclus dans le deuxième sous-intervalle, appelée deuxième agrégation (M2i),
- identifier (340) un écart maximum (Δmax) parmi les écarts calculés,
- en réponse à la détermination que la deuxième agrégation (M2) associée à l'écart maximum (Δmax) n'appartient pas à une plage de sensibilité (S), générer (350) un signal d'alerte.

2. Dispositif de génération d'alerte selon la revendication 1, dans lequel la fonction d'agrégation est choisie parmi : une moyenne, une médiane, un écart-type, un maximum, un minimum**.**

3. Dispositif de génération d'alerte selon la revendication 1 ou 2, dans lequel le dispositif de génération d'alerte (150) comprend un serveur (130), un terminal mobile (120) ou un dispositif de mesure (110).

4. Dispositif de génération d'alerte (150) selon l'une quelconque des revendications précédentes, dans lequel le paramètre physiologique est choisi parmi : une température du poignet, une température corporelle, une fréquence cardiaque nocturne, une fréquence cardiaque au repos, une saturation en oxygène du sang (SpO2), un indice d'apnée-hypopnée (AHI), un poids ou une masse, une masse graisseuse (en poids ou en pourcentage), un masse musculaire (en poids ou en pourcentage), une vitesse de l'onde de pouls (VOP), une variabilité de la fréquence cardiaque (VFC), un nombre de pas quotidien, une activité sportive, une durée du sommeil, un score de qualité de sommeil, une heure du coucher, une heure du lever, la fréquence respiratoire, la pression ou tension artérielle, un niveau d'un composé présent dans l'urine.

5. Dispositif de génération d'alerte (150) selon l'une quelconque des revendications précédentes, dans lequel l'intervalle de temps de détection (Pd) présente une durée prédéfinie associée au paramètre physiologique.

6. Dispositif de génération d'alerte (150) selon l'une quelconque des revendications précédentes, dans lequel :
- le dispositif de génération d'alerte (150) est configuré pour mettre en œuvre la méthode de génération d'alerte de manière répétée, et/ou
- le dispositif de génération d'alerte (150) est configuré pour œuvre la méthode de génération d'alerte à chaque réception de donnée, l'intervalle de temps de détection (Pd) défini comprenant un horodatage associé à la dernière donnée reçue.

7. Dispositif de génération d'alerte (150) selon l'une quelconque des revendications précédentes, dans lequel chaque sous-intervalle (P1i, P2i) présente une durée minimale, et dans lequel :
- la durée minimale de chaque sous-intervalle (P1i, P2i) est fonction de la durée de l'intervalle de temps de détection (Pd), et/ou
- la durée minimale de chaque sous-intervalle (P1i, P2i) est fonction du paramètre physiologique.

8. Dispositif de génération d'alerte (150) selon l'une quelconque des revendications précédentes, dans lequel le dispositif de génération d'alerte (150) est configuré pour définir toutes les partitions possibles de l'intervalle de temps de détection (Pd) en deux sous-intervalles (P1i, P2i) continus.

9. Dispositif de génération d'alerte (150) selon l'une quelconque des revendications précédentes, dans lequel la plage de sensibilité (S) est fonction du paramètre physiologique.

10. Dispositif de génération d'alerte (150) selon l'une quelconque des revendications précédentes, dans lequel le dispositif de génération d'alerte (150) est en outre configuré pour recevoir une série temporelle de données physiologiques sur un intervalle de temps de sensibilité (Ps), chaque donnée physiologique de la série temporelle étant associée à un horodatage inclus de l'intervalle de temps de sensibilité (Ps), la plage de sensibilité (S) étant fonction de de l'historique des données du paramètre physiologique sur l'intervalle de temps de sensibilité (Ps).

11. Dispositif de génération d'alerte (150) selon la revendication 10, dans lequel l'intervalle de temps de sensibilité (Ps) présente une durée plus longue que l'intervalle de temps de détection (Pd).

12. Dispositif de génération d'alerte (150) selon la revendication 10 ou 11, dans lequel l'intervalle de temps de sensibilité (Ps) et le deuxième sous-intervalle (Pd2) sont disjoints.

13. Dispositif de génération d'alerte (150) selon l'une quelconque des revendications précédentes, dans lequel le dispositif de génération d'alerte (150) est configuré pour générer un message d'alerte à partir du signal d'alerte.

14. Dispositif de génération d'alerte (150) selon la revendication 13, dans lequel le dispositif de génération d'alerte (150) est configuré pour mettre en œuvre un grand modèle linguistique avec au moins la partition associée à l'écart maximum (Δmax) et les agrégations associées en entrées suite à la génération d'un signal d'alerte pour générer un message d'alerte.

15. Dispositif de génération d'alerte (150) selon l'une quelconque des revendications précédentes, dans lequel la méthode de génération d'alerte comprend en outre :
- extraire de la partition associée à l'écart maximum (Δmax) un marqueur temporel diviseur (E) qui partitionne l'intervalle de temps (P) en les deux sous-intervalles (P1i, P2i) continus,
- générer une notification d'évènement temporel, l'évènement temporel étant associé au marqueur temporel diviseur extrait (E).

16. Dispositif de génération d'alerte (150) selon l'une quelconque des revendications précédentes, dans lequel le dispositif de génération d'alerte (150) est configuré pour mettre en œuvre la méthode de génération d'alerte pour une pluralité de paramètres physiologiques, notamment sur le même intervalle de temps de détection (Pd).
